# EUROPÄISCHE PATENTSCHRIFT

(11) **EP 0 562 377 B1**
(45) Veröffentlichungstag und Bekanntmachung des Hinweises auf die Patenterteilung: **14.05.1997**
(21) Anmeldenummer: 93103911.9
(22) Anmeldetag: 11.03.1993
(51) Int. Cl.: C07D 261/18

(54) **Herstellung von Isoxazol-3-carbonsäureamid-4-carbonsäureestern durch selektive Amidierung**
Preparation of isoxazole-3-carboxamide-4-carboxylic acid esters by selective amidation
Préparation d'esters d'acides 3-carboxamido-Isoxazole-4-carboxyliques par amidation sélective

(30) Priorität: 26.03.1992 DE 4209849
(43) Veröffentlichungstag der Anmeldung: 29.09.1993
(73) Patentinhaber: BASF Aktiengesellschaft, 67063 Ludwigshafen (DE)
(72) Erfinder: Maywald, Volker, Dr., W-6700 Ludwigshafen (DE); Kuekenhoehner, Thomas, Dr., W-6737 Boehl-Iggelheim (DE); Hamprecht, Gerhard, Dr., W-6940 Weinheim (DE)

(56) Entgegenhaltungen:
- EP-A- 0 418 667

## Beschreibung

Die vorliegende Erfindung betrifft die Herstellung von Isoxazol-3-carbonsäureamid-4-carbonsäureestern der Formel I 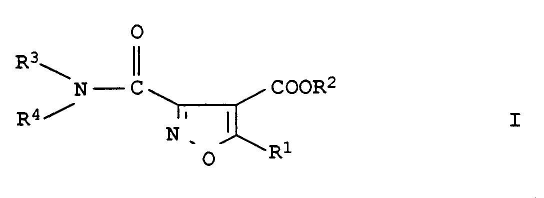 in der die Substituenten die folgende Bedeutung haben:
- R¹: Wasserstoff, Alkyl, Cycloalkyl, Phenyl oder ein 5- bis 6-gliedriger heterocyclischer Rest, wobei die organischen Reste unter den Reaktionsbedingungen inerte Substituenten tragen können;
- R²: Alkyl, Cycloalkyl, Benzyl oder ein C₃-C₆-Alkenylrest;
- R³: Wasserstoff, Alkyl, Cycloalkyl und
- R⁴: ein aliphatischer, cycloaliphatischer oder gegebenenfalls substituierter Phenylrest oder R⁴ zusammen mit R³ eine 4- bis 7-gliedrige Alkylenkette, die durch Sauerstoff, Schwefel oder N-Methyl unterbrochen sein kann.

Isoxazol-3-carbonsäureamid-4-carbonsäurederivate I sind Zwischenprodukte für organische Synthesen, insbesondere zur Herstellung von Pflanzenschutzmitteln (vgl. DE-A-39 31 627). Der vorliegenden Erfindung lag deshalb die Aufgabe zugrunde, ein möglichst einfaches und wirtschaftliches Verfahren zur Synthese der Verbindungen I finden.

Demgemäß wurde ein Verfahren zur Herstellung von Isoxazol-3-carbonsäureamid-4-carbonsäureestern I gefunden, das dadurch gekennzeichnet ist, daß man einen Isoxazol-3,4-dicarbonsäurediester der Formel II 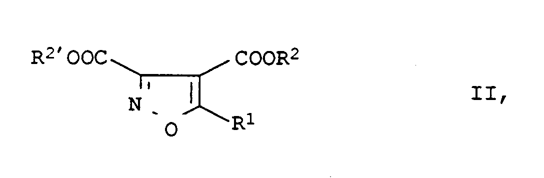 in der R^{2'} die Bedeutung von R² hat, und gleich oder verschieden von R² ist, mit einem primären oder sekundären Amin der Formel III 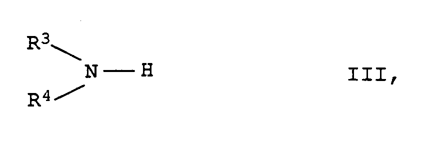 in der R³ und R⁴ die obengenannte Bedeutung haben, in Gegenwart oder Abwesenheit eines Lösungsmittels selektiv an der Estergruppe in 3-Position amidiert.

Das erfindungsgemäße Verfahren besticht durch die Einfachheit des gewählten Lösungsweges, von dem keineswegs zu erwarten gewesen war, daß er zum Erfolg führen würde, da zwei gleiche funktionelle Gruppen am Isoxazolsystem II vorliegen, die beide mit dem Amin III reagieren sollten.

Überraschenderweise wurde gefunden, daß die Amidierung praktisch ausschließlich an der Estergruppe in 3-Position stattfindet. Die entsprechenden Isoxazol-4-carbonsäureamid-Derivate IV 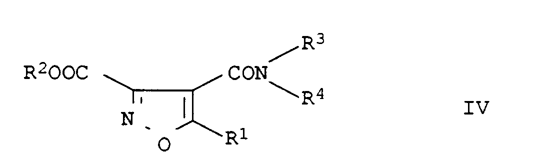 bzw. die Isoxazol-3,4-dicarbonsäurediamide V, 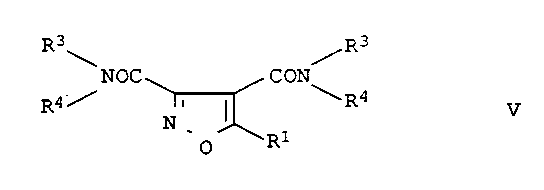 deren Bildung bei der erfindungsgemäßen Umsetzung in beträchtlichem Außmaß zu erwarten war, werden unter den angewandten Reaktionsbedingungen nicht oder nur in vernachlässigbar geringem Ausmaß gebildet.

Nach dem erfindungsgemäßen Verfahren können die Isoxazol-3,4-dicarbonsäure-Diester II mit praktisch allen primären bzw. sekundären Aminen der Formeln IIIa (R³ = H) bzw. IIIb (R³ ≠ H)

H₂N―R⁴ IIIa

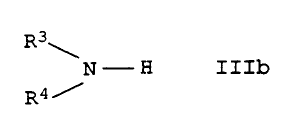

selektiv zu den entsprechenden Isoxazol-3-amid-4-carbonsäureestern II umgesetzt werden.

Vorzugsweise werden Amine III eingesetzt, in denen R³ für Wasserstoff, C₁-C₆-Alkyl, insbesondere C₁-C₄-Alkyl wie Methyl, Ethyl, Propyl, Isopropyl, Butyl, Isobutyl, sek.-Butyl, tert.-Butyl und C₃-C₆-Cycloalkyl wie Cyclopropyl, Cyclobutyl, Cyclopentyl und Cyclohexyl, insbesondere Cyclopropyl steht und R⁴ einen gegebenenfalls substituierten Alkyl-, Cycloalkyl-, Alkenyl- oder Alkinylrest bedeutet. Beispielsweise seien folgende Reste R⁴ aufgeführt: C₁-C₆-Alkyl, insbesondere C₁-C₄-Alkyl wie unter R³ genannt, das durch Cyclopropyl, Halogen wie Fluor, Chlor, Brom, Cyano oder C₁-C₆-Alkoxy substituiert sein kann, C₃-C₆-Cycloalkyl, das durch C₁-C₄-Alkyl oder Halogen substituiert sein kann, C₃-C₆-Alkenyl oder C₃-C₆-Alkinyl. Des weiteren werden vorzugsweise Amine III verwendet, in denen die Reste R³ und R⁴ gemeinsam eine C₄-C₇-Methylenkette, welche durch Sauerstoff, Schwefel oder N-Methyl unterbrochen sein kann, bilden.

Besonders bevorzugt sowohl im Hinblick auf bestimmungsgemäße Verwendung als auch aus verfahrenstechnischer Sicht werden die Isoxazol-3,4-dicarbonsäurediester II mit einfachen primären aliphatischen Aminen wie Methylamin, Ethylamin, Propylamin, Isopropylamin, Butylamin, Isobutylamin, sek-Butylamin, (R)-sek-Butylamin, (S)-sek-Butylamin, tert-Butylamin, Neopentylamin und 1,1-Dimethylpropylamin,
substituierten primären aliphatischen Aminen wie 1-Cyclopropylethylamin, (R)-1-Cyclopropylethylamin, (S)-1-Cyclopropylethylamin und Cyclopropylmethylamin,
Cycloalkylaminen wie Cyclopropylamin, Cyclobutylamin, Cyclopentylamin, Cyclohexylamin, 1-Methylcyclopropylamin,
ungesättigten Aminen wie Prop-2-enylamin, 1-Methylprop-2-enylamin, 1,1-Dimethyl-prop-2-enylamin, Prop-2-inylamin, 1-Methylprop-2-inylamin und 1,1-Dimethylprop-2-inylamin,
einfachen sekundären Aminen wie Dimethylamin, Methylethylamin, Diethylamin, Dipropylamin, Dibutylamin, Methylisopropylamin, Diisopropylamin und Methyl-tert-butylamin,
sowie cyclischen sekundären Aminen wie Pyrrolidin, Piperidin und Morpholin umgesetzt.

Entsprechend den unterschiedlichen Verfahrensweisen kann das Amin III in stöchiometrischer Menge als auch in praktisch beliebigem, molaren Überschuß bezüglich des Reaktanden II eingesetzt werden. Vorteilhaft wird das Amin III bezüglich des Diesters jedoch in der 1- bis 8-fach, insbesondere 1- bis 5-fach molaren Menge angewandt. Es kann jedoch auch vorteilhaft sein, das Amin III so zur vorgelegten Verbindung II zuzugeben, daß praktisch während des gesamten Reaktionsverlaufs eine unterstöchiometrische Menge des Amins III bezüglich des Isoxazol-3,4-dicarbonsäure-diesters II vorliegt.

Bei einer besonders bevorzugten Arbeitsweise des erfindungsgemäßen Verfahrens wird das zur Amidierung verwendete Amin III im Überschuß, bezogen auf den Isoxazol-3,4-dicarbonsäure-diester II verwendet, wobei das überschüssige Amin III selbst als Lösungsmittel fungiert.

Die erfindungsgemäße Umsetzung läßt sich gegebenenfalls in einem unter den Reaktionsbedingungen inerten Lösungsmittel durchführen. Vorzugsweise verwendete Lösungsmittel sind Ether wie Diethylether, Diisopropylether, Methyl-tert.-butylether, Dimethoxyethan, Diethylenglykoldimethylether, Tetrahydrofuran oder Dioxan, Halogenkohlenwasserstoffe wie Dichlormethan, Chloroform oder Chlorbenzol, aromatische Kohlenwasserstoffe wie Benzol, Toluol oder Xylol sowie Sulfoxide wie Dimethylsulfoxid oder Sulfolan. Besonders bevorzugte Lösungsmittel sind Alkohole, z.B. Alkanole oder Cycloalkanole wie n-Butanol, Isobutanol, tert.-Butanol, Glykol, Glycerin, n-Propanol, Isopropanol, Amylalkohol, Cyclohexanol, 2-Methyl-4-pentanol, Ethylenglykolmonoethylether, 2-Ethylhexanol, Methylglykol und insbesondere Ethanol, Methanol. Es können selbstverständlich auch Lösungsmittelgemische eingesetzt werden.

Zweckmäßigerweise verwendet man die 0,5- bis 10-fache, vorzugsweise 1- bis 5-fache Gewichtsmenge an Lösungsmittel bezogen auf II.

Im erfindungsgemäßen Verfahren wird der als Ausgangsverbindung benutzte Isoxazol-3,4-dicarbonsäure-diester II mit dem Amin III bei Temperaturen von 0 bis 100°C, besonders bevorzugt bei 20 bis 80°C, umgesetzt. Dabei wird zweckmäßigerweise die Umsetzung sterisch nicht gehinderter Amine bei Temperaturen von 20 bis 60°C durchgeführt, wohingegen bei Verwendung von Aminen III mit einem oder zwei sterisch anspruchsvollen Substituenten R³ und R⁴ im allgemeinen bei höheren Temperaturen, insbesondere bei 60 bis 100°C, gearbeitet wird.

Das erfindungsgemäße Verfahren wird im allgemeinen bei Atmosphärendruck ausgeführt. Je nach Art des verwendeten Amins oder Lösungsmittels, kann es sich jedoch als besonders vorteilhaft auswirken, wenn die Umsetzung unter erhöhtem Druck, insbesondere unter autogen erhöhtem Druck in einem Autoklaven durchgeführt wird. Diese Maßnahme führt insbesondere dann zu vorteilhaften Ergebnissen, wenn niedrigsiedende Amine III, wie Methylamin, Ethylamin, Isopropylamin oder Cyclopropylamin oder niedrigsiedende Lösungsmittel wie Diethylether oder Methanol verwendet werden. Selbstverständlich kann das erfindungsgemäßen Verfahren auch unter exogen erhöhtem Druck betrieben werden, zweckmäßigerweise im Druckbereich von 1 bis 50 bar.

Die Amidierung kann sowohl auf konventionelle Weise diskontinuierlich in Rührkesseln oder Rührautoklaven als auch kontinuierlich in Rührkesselkaskaden oder Rohrreaktoren betrieben werden. Insgesamt weist das Verfahren keine verfahrenstechnischen Besonderheiten auf, so daß sich weitere Angaben hierzu erübrigen. Je nach den angewandten Verfahrensbedingungen wie Druck und Temperatur und je nach den verwendeten Ausgangsverbindungen ist die Umsetzung im allgemeinen nach 1 bis 20 Stunden Reaktionsdauer beendet. Da die gewünschten Isoxazol-3-carbonsäureamid-derivate nach dem erfindungsgemäßen Verfahren mit einer hohen Selektivität gebildet werden, genügt es in vielen Fällen zur Aufarbeitung einfach das Lösungsmittel und/oder das überschüssige Amin III abzudestillieren, um ein für die Weiterverarbeitung genügend reines Produkt I zu erhalten. Es ist aber auch möglich, das Reaktionsgemisch auf herkömmliche Weise aufzuarbeiten, indem man das überschüssige Amin mit verdünnten, wäßrigen Säuren extrahiert und das Produkt I aus der organischen Phase isoliert. Diese Verfahrensweise wird insbesondere dann bevorzugt angewendet, wenn das abzutrennende Amin III relativ schwerflüchtig ist.

Im Hinblick auf die bestimmungsgemäße Verwendung der Isoxazolderivate I werden nach dem erfindungsgemäßen Verfahren vorzugsweise Isoxazol-3,4-dicarbonsäurediester der allgemeinen Formel II, in der die Reste R¹ und R² die folgende Bedeutung haben, zu den entsprechenden Isoxazol-3-carbonsäureamid-4-carbonsäureestern I umgesetzt:
- R¹: Wasserstoff,
ein niedermolekularer Alkylrest wie C₁-C₆-Alkyl, insbesondere C₁-C₄-Alkyl wie Methyl, Ethyl, Propyl, Isopropyl, Butyl, Isobutyl, sek.-Butyl, tert.-Butyl, das durch ein bis drei Halogenatome wie Fluor, Chlor, Brom, Iod, insbesondere Fluor und Chlor, einen C₁-C₃-Alkoxyrest wie Methoxy, Ethoxy, Propoxy, Isopropoxy, insbesondere Methoxy oder einen C₃-C₆-Cycloalkylrest wie Cyclopropyl, Cyclobutyl, Cyclopentyl und Cyclohexyl, insbesondere Cyclopropyl, substituiert sein kann,
ein Cycloalkylrest wie C₃-C₈-Cycloalkyl, z.B. Cyclopropyl, Cyclobutyl, Cyclopentyl, Cyclohexyl, Cycloheptyl, Cyclooctyl, insbesondere Cyclopropyl, das ein bis dreimal durch C₁-C₄-Alkyl wie vorstehend genannt, insbesondere Methyl substituiert sein kann,
ein 5- bis 6-gliedriger gesättigter, ungesättigter oder aromatischer heterocyclischer Rest, enthaltend ein oder zwei Heteroatome, ausgewählt aus der Gruppe Sauerstoff, Schwefel und Stickstoff wie 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 2-Oxazolyl, 4-Oxazolyl, 5-Oxazolyl, 2-Thiazolyl, 4-Thiazolyl, 5-Thiazolyl, 2-Pyridyl, 3-Pyridyl, 4-Pyridyl, der durch C₁-C₃-Alkyl wie vorstehend genannt, insbesondere Methyl, C₁-C₃-Alkoxy wie vorstehend genannt, insbesondere Methoxy oder Halogen wie Fluor, Chlor, Brom und Iod, insbesondere Fluor und Chlor, substituiert sein kann;
Phenyl, das eine bis drei der folgenden Gruppen tragen kann: C₁-C₄-Alkyl wie vorstehend genannt, insbesondere Methyl, C₁-C₄-Halogenalkyl, insbesondere Trifluormethyl, C₁-C₄-Alkoxy, insbesondere Methoxy, C₁-C₄-Halogenalkoxy, insbesondere Trifluormethoxy, Halogen wie vorstehend genannt, insbesondere Fluor und Chlor, Nitro und Cyano;
- R², R^{2'}: C₁-C₄-Alkyl wie unter R¹ genannt, insbesondere Methyl und Ethyl, C₃-C₆-Cycloalkyl wie unter R¹ genannt, insbesondere Cyclohexyl, Benzyl und C₃-C₆-Alkenyl, insbesondere Allyl.

### Beispiele

### Beispielvorschriften zur selektiven Umsetzung von Isoxazol-3,4-dicarbonsäuredialkylestern II mit Aminen III

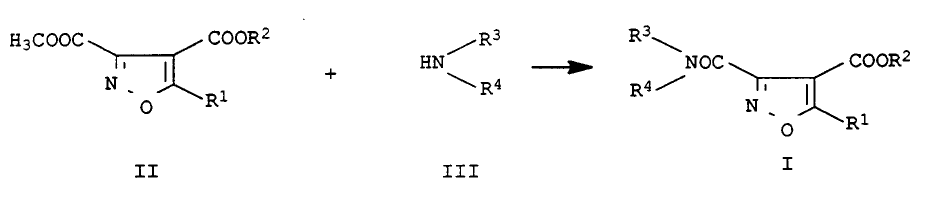

a) in Gegenwart eines Lösungsmittels
b) in Abwesenheit eines Lösungsmittels
a) Zu einer Lösung von 0,3 mol 5-Alkyl-isoxazol-3,4-dicarbonsäuredialkylester I in 100 ml trockenem Alkohol werden bei 25°C 0,6 mol Cyclopropylamin zugetropft, anschließend wird 12 h bei Raumtemperatur nachgerührt. Nach Beendigung der Reaktion werden das überschüssige Cyclopropylamin und das Solvens im Vakuum abgezogen. In analoger Weise können andere primäre oder sekundäre Amine umgesetzt werden. Die Versuchsergebnisse sind in der Tabelle zusammengestellt.

**Tabelle**

| Beispiel Nr. | R¹ | R² | R³ | R⁴ | Lösungsmittel | Ausbeute (%) | Fp [°C]; ¹H-NMR (CDCl₃); 250 MHz; δ in ppm |
|---|---|---|---|---|---|---|---|
| 1 | Me | Me | H | cyclo-Pr | MeOH | 74 | 0,60-0,92 (m,4H); 2,70 (s,3H); 2,93 (m,1H); 3,92 (s,3H); 8,27 (bs,1H,NH) |
| 2 | Et | Me | H | cyclo-Pr | MeOH | 93 | 0,58-0,95 (m,4H); 1,34 (t,3H); 2,93 (m,1H); 3,10 (q,2H); 3,92 (s,3H); 7,95 (bs,1H,NH) |
| 3 | iPr | Me | H | cyclo-Pr | MeOH | 87 | 0,60-0,93 (m,4H); 1,36 (d, 6H); 2,92 (m,1H); 3,70 (sp,1H); 3,89 (s,3H); 7,84 (bs,1H,NH) |
| 4 | iPr | Et | H | cyclo-Pr | EtOH | 85 | 0,60-0,90 (m,4H); 1,36 (d,6H); 1,37 (t,3H); 2,93 (m,1H); 3,73 (sp,1H); 4,35 (q,2H); 7,75 (bs,1H,NH) |
| Me = Methyl, Et = Ethyl, Pr = Propyl | | | | | | | |

b) 32,0 g (0,15 mol) 5-Ethyl-isoxazol-3,4-dicarbonsäuredimethylester und 44,3 g (0,75 mol) Methylethylamin werden in einem 300 ml Miniautoklav 6 Stunden auf 60°C erhitzt. Anschließend wird das Reaktionsgemisch am Rotationsverdampfer eingeengt, in Ether aufgenommen und zweimal mit je 50 ml 1n HCl extrahiert. Die organische Phase wird über Magnesiumsulfat getrocknet und das Solvens im Vakuum abgezogen. Man erhält 30,1 g (84 %) 5-Ethyl-3-methylethylcarbamoyl-isoxazol-4-carbonsäuremethylester als Gemisch aus zwei Amid-Rotameren. ¹H-NMR (CDCl₃; 250 MHz) δ=1,17 und 1,27 (2t; 2x3H), 1,35 (t; 3H), 2,89 und 3,12 (2s; 2x3H), 3,18 (q; 2H), 3,25 und 3,63 (2q; 2x2H), 3,84 (s; 3H).

Bei der gaschromatographischen Untersuchung der Reaktionsmischungen vor der Aufarbeitung konnte in den beschriebenen Versuchen kein Isoxazol-4-carbonsäureamid IV nachgewiesen werden. Die Menge an Diamid V lag bei maximal 3 Prozent.

## Patentansprüche

1. Verfahren zur Herstellung von Isoxazol-3-carbonsäureamid-4-carbonsäureestern der Formel I 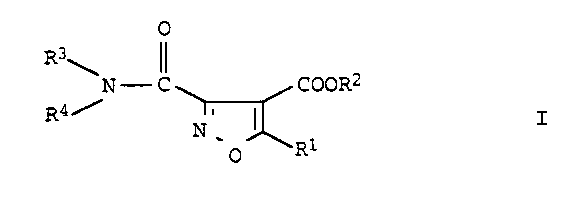 in der die Substituenten die folgende Bedeutung haben:
R¹ Wasserstoff, Alkyl, Cycloalkyl, Phenyl oder ein 5- bis 6-gliedriger heterocyclischer Rest, wobei die organischen Reste unter den Reaktionsbedingungen inerte Substituenten tragen können;
R² Alkyl, Cycloalkyl, Benzyl oder ein C₃-C₆-Alkenylrest;
R³ Wasserstoff, Alkyl, Cycloalkyl und
R⁴ ein aliphatischer, cycloaliphatischer oder gegebenenfalls substituierter Phenylrest oder R⁴ zusammen mit R³ eine 4- bis 7-gliedrige Alkylenkette, die durch Sauerstoff, Schwefel oder N-Methyl unterbrochen sein kann; dadurch gekennzeichnet, daß man einen Isoxazol-3,4-dicarbonsäurediester der Formel II 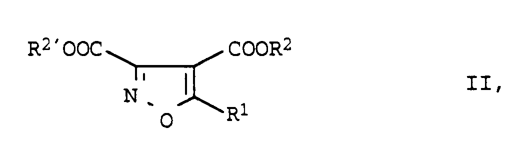 in der R^{2'} die Bedeutung von R² hat, und gleich oder verschieden von R² ist, mit einem primären oder sekundären Amin der Formel III 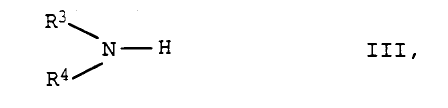 in der R³ und R⁴ die obengenannte Bedeutung haben,
in Gegenwart oder Abwesenheit eines Lösungsmittels selektiv an der Estergruppe in 3-Position amidiert.

2. Verfahren nach Anspruch 1, dadurch gekennzeichnet, daß man ein primäres Amin III zur Amidierung verwendet.

3. Verfahren nach Anspruch 1, dadurch gekennzeichnet, daß man das Amin in einer Menge von 1 bis 5 mol, bezogen auf den Diester II verwendet.

4. Verfahren nach Anspruch 1, dadurch gekennzeichnet, daß man die Amidierung in Abwesenheit eines Lösungsmittels und mit einem Überschuß an Amin, bezogen auf II, vornimmt.

5. Verfahren nach Anspruch 1, dadurch gekennzeichnet, daß man die Amidierung in einem niedermolekularen Alkanol als Lösungsmittel vornimmt.

## Claims

1. A process for preparing 3-carbamoylisoxazole-4-carboxylic esters of the formula I 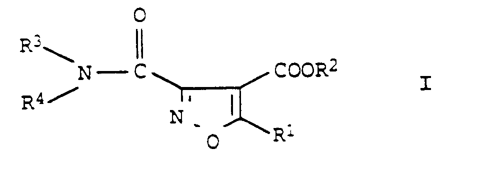 where
R¹ is hydrogen, alkyl, cycloalkyl, phenyl or a 5- to 6-membered heterocyclic radical, it being possible for the organic radicals to carry substituents which are inert under the reaction conditions;
R² is alkyl, cycloalkyl, benzyl or C₃-C₆-alkenyl;
R³ is hydrogen, alkyl or cycloalkyl and
R⁴ is an aliphatic or cycloaliphatic radical or unsubstituted or substituted phenyl, or R⁴ together with R³ is a 4- to 7-membered alkylene chain which can be interrupted by oxygen, sulfur or N-methyl;
which comprises selective amidation at position 3 of an isoxazole-3,4-dicarboxylic diester of the formula II 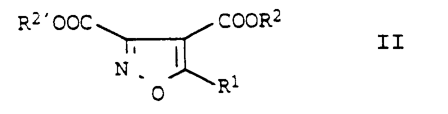 where R^{2'} has the same meanings as R² and is identical to or different from R², with a primary or secondary amine of the formula III 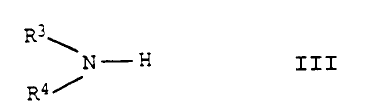 where R³ and R⁴ have the abovementioned meanings,
in the presence or absence of a solvent.

2. A process as claimed in claim 1, wherein a primary amine III is used for the amidation.

3. A process as claimed in claim 1, wherein the amine is used in an amount of from 1 to 5 mol per mol of diester II.

4. A process as claimed in claim 1, wherein the amidation is carried out in the absence of a solvent and using an excess of amine relative to II.

5. A process as claimed in claim 1, wherein the amidation is carried out in a lower alkanol as solvent.

## Revendications

1. Procédé pour la préparation d'esters isoxazole-3-carboxamide-4-carboxyliques de formule I 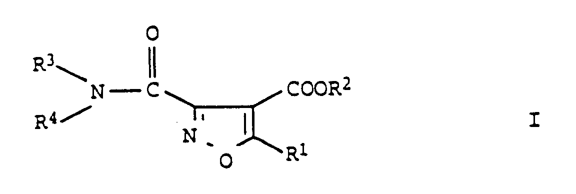 dans laquelle les symboles ont les significations suivantes :
R¹ représente l'hydrogène, un groupe alkyle, cycloalkyle, phényle ou un groupe hétérocyclique à cinq ou six chaînons, les groupes organiques pouvant porter des substituants inertes dans les conditions de la réaction ;
R² représente un groupe alkyle, cycloalkyle, benzyle ou alcényle en C3-C6 ;
R³ représente l'hydrogène, un groupe alkyle, cycloalkyle et
R⁴ représente un groupe phényle aliphatique, cycloaliphatique ou éventuellement substitué ou bien R⁴ et R³ forment ensemble une chaîne alkylène de quatre à sept chaînons qui peut être interrompue par l'oxygène, le soufre ou un groupe N-méthyle ;
caractérisé par le fait que l'on amide sélectivement sur le groupe ester en position 3 et en présence ou non d'un solvant, un diester isoxazole-3,4-dicarboxylique de formule II 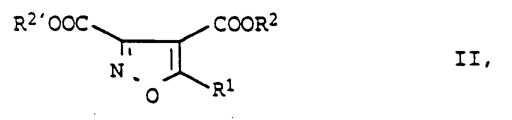 dans laquelle R^{2'} a les mêmes significations que R² mais peut avoir une signification identique ou différente de celle de R², par une amine primaire ou secondaire de formule III 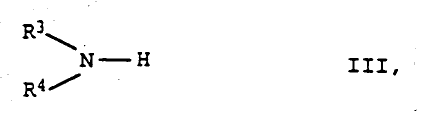 dans laquelle R³ et R⁴ ont les significations indiquées ci-dessus.

2. Procédé selon la revendication 1, caractérisé par le fait que l'on utilise pour l'amidation une amine primaire III.

3. Procédé selon la revendication 1, caractérisé par le fait que l'on utilise l'amine en quantité de une à cinq moles par rapport au diester II.

4. Procédé selon la revendication 1, caractérisé par le fait que l'on procède à l'amidation en l'absence de solvant mais avec un excès de l'amine par rapport à II.

5. Procédé selon la revendication 1, caractérisé par le fait que l'on procède à l'amidation dans un solvant consistant en un alcanol à bas poids moléculaire.
